# FASCICULE DE BREVET EUROPEEN

(11) **EP 1 562 514 B1**
(45) Date de publication et mention de la délivrance du brevet: **08.08.2007**
(21) Numéro de dépôt: 03773818.4
(22) Date de dépôt: 06.10.2003
(51) Int. Cl.: A61F 2/00

(54) **RENFORT PARIETAL ANATOMIQUE POUR LE TRAITEMENT D'UNE HERNIE INGUINALE**
ANATOMISCHES VERSTÄRKUNGSIMPLANTAT FÜR DIE BAUCHWAND ZUR BEHANDLUNG EINER INGUINALHERNIE
ANATOMICAL PARIETAL REINFORCEMENT FOR TREATING INGUINAL HERNIA

(30) Priorité: 07.10.2002 US 265395
(43) Date de publication de la demande: 17.08.2005
(73) Titulaire: SOFRADIM PRODUCTION, 01600 Trévoux (FR)
(72) Inventeur: THERIN, Michel, F-69004 Lyon (FR); WILSON, Russel, Peachtree City, GA 30269 (US); RAMSHAW, Bruce, M., D., Peachtree City, GA 30269 (US)
(74) Mandataire: Brédeville, Odile Marie
(86) Numéro de dépôt international: PCT/FR2003/002931
(87) Numéro de publication internationale: WO 2004/032797

(56) Documents cités:
- FR-A- 2 807 937
- US-A- 6 066 777
- US-A1- 2002 029 063

## Description

La présente invention concerne un renfort pariétal anatomique pour le traitement d'une hernie inguinale, notamment par voie laparoscopique, dont l'état de la technique le plus proche est défini par le document US-A-6066777.

Une hernie inguinale résulte du passage du péritoine, accompagné ou non de certains viscères, au travers du canal inguinal qu'emprunte le cordon spermatique (hernie dite alors indirecte ou oblique externe) ou au travers du canal fémoral qu'empruntent les vaisseaux iliaques (hernie dite alors fémorale), ou au travers d'une zone faible de la paroi abdominale inguinale située médialement par rapport aux vaisseaux épigastriques (hernie dite alors directe).

Pour le traitement d'une telle hernie, il est connu d'utiliser un renfort de paroi abdominale formé par une ou plusieurs pièces prothétiques poreuses en matériau ajouré biocompatible, ce renfort étant placé autour de l'orifice interne du canal inguinal, entre le péritoine et les muscles abdominaux.

Un renfort "anatomique" connu comprend une première pièce et une deuxième pièce assemblées l'une à l'autre.

La première pièce présente une forme en "L" renversé comprenant une partie principale destinée à venir reposer contre la paroi musculaire, notamment les muscles grand droit et transverse, et une partie secondaire, minoritaire en surface, destinée à venir recouvrir l'extrémité supérieure de l'os pubien et le ligament de Cooper. Le bord longitudinal de la partie principale à partir duquel se projette ladite partie secondaire a une forme plus ou moins ondulée, adaptée à celle des structures inguinales inférieures, à savoir notamment les vaisseaux spermatiques et iliaques et le muscle psoas.

La deuxième pièce est assemblée à la première pièce le long d'une partie ou de la totalité de ce bord longitudinal et du bord latéral de ladite partie secondaire consécutif à ce bord longitudinal, et est destinée à épouser la forme générale desdites structures inguinales inférieures, c'est à dire les vaisseaux iliaques et le muscle psoas latéralement.

Dans certaines techniques chirurgicales, le cordon spermatique doit passer au travers de ce renfort et, pour permettre ce passage, un renfort existant comprend un trou aménagé dans ladite première pièce et une découpe reliant ce trou à un bord périphérique de cette première pièce, cette découpe pouvant être délimitée par deux rabats se chevauchant. Lorsque ladite deuxième pièce est reliée à ladite première pièce sur une partie seulement dudit bord longitudinal, le cordon spermatique peut être engagé entre ladite première pièce et ladite deuxième pièce jusqu'à prendre place dans une encoche aménagée dans ladite première pièce ou dans ladite deuxième pièce, ou dans ces deux pièces.

Ces trous ou encoches conduisent à découper le matériau ajouré formant l'une et/ou l'autre desdites pièces et ont ainsi pour inconvénient de former une zone relativement lâche autour du cordon spermatique, et donc autour de l'orifice interne du canal inguinal. Il résulte un renforcement limité de la paroi abdominale à cet endroit, pouvant rendre possible une récidive de hernie.

En outre, ces trous ou encoches impliquent que le renfort soit mis en place selon une position déterminée, définie par la position du cordon spermatique, ce qui peut conduire à des difficultés pour réaliser cette mise en place.

La présente invention vise à remédier à ces inconvénients.

Un objectif de l'invention est donc de fournir un renfort pariétal anatomique pour le traitement d'une hernie inguinale qui permette de prévenir efficacement toute récidive de hernie, particulièrement autour du cordon spermatique dans le cas des prothèses fendues.

Un autre objectif de l'invention est de fournir un renfort n'impliquant pas un positionnement déterminé de ce renfort pour permettre le passage du cordon spermatique au travers de ce renfort.

Un objectif supplémentaire de l'invention est de faciliter l'engagement du cordon spermatique au travers du renfort.

Le renfort selon l'invention comprend une première pièce et une deuxième pièce assemblées l'une à l'autre, ladite première pièce présentant une partie principale destinée à venir reposer contre la paroi musculaire, notamment les muscles grand droit et transverse, et une partie secondaire, minoritaire en surface, destinée à venir recouvrir l'extrémité supérieure de l'os pubien et le ligament de Cooper, le bord longitudinal de ladite partie principale duquel se projette ladite partie secondaire et le bord latéral de cette partie secondaire consécutif à ce bord longitudinal ayant une forme plus ou moins ondulée, adaptée à celle des structures inguinales inférieures, à savoir notamment les vaisseaux spermatiques et iliaques et le muscle psoas, ladite deuxième pièce étant assemblée à ladite première pièce le long d'une partie ou de la totalité dudit bord longitudinal et dudit bord latéral, et étant destinée à épouser la forme générale desdites structures inguinales inférieures.

Dans ce renfort, ladite première pièce comprend une découpe aménageant une zone de passage du cordon spermatique à travers elle située à distance dudit bord longitudinal, et comprend un rabat relié à elle, dimensionné de manière à s'étendre près dudit bord longitudinal et à recouvrir largement la zone de ladite première pièce s'étendant entre ledit bord longitudinal et ladite zone de passage, ce rabat pouvant être soulevé par rapport à ladite première pièce pour l'engagement du cordon spermatique entre ladite première pièce et lui-même et pouvant être rabattu contre ladite première pièce pour maintenir ce cordon entre cette première pièce et lui-même.

Les zones par lesquelles le cordon spermatique rentre au travers du renfort et sort au-delà de ce renfort sont ainsi mutuellement décalées et distantes, de sorte que le cordon spermatique ne traverse pas le renfort selon un parcours direct, sensiblement perpendiculaire à ladite première pièce, mais selon un parcours en chicane. Ce parcours se fait de plus entre ladite première pièce d'une part et ledit rabat d'autre part, donc entre deux épaisseurs de matériau.

Cette configuration de parcours et ces deux épaisseurs de matériau éliminent toute découpe d'un orifice dans ladite première pièce destiné à être traversé directement par le cordon spermatique, risquant de former une zone plus ou moins lâche de cette première pièce autour du cordon spermatique. Dans le renfort selon l'invention, au contraire, le cordon rentre dans le renfort en étant reçu contre ladite zone de la première pièce s'étendant entre ledit bord longitudinal et ladite zone de passage, et sort du renfort par ladite zone de passage, au niveau de laquelle il est parfaitement recouvert par ledit rabat.

Ce renfort permet ainsi de parfaitement prévenir toute récidive de hernie.

De plus, ledit rabat ne détermine pas une zone précise d'engagement du cordon spermatique au travers du renfort, et n'oblige donc pas à un positionnement déterminé de ce renfort pour permettre ce passage. La mise en place du renfort s'en trouve facilitée, et le rabat facilite lui-même l'engagement du cordon spermatique au travers du renfort.

La largeur de ladite zone de la première pièce s'étendant entre ledit bord longitudinal et ladite zone de passage peut aller de 0,3 à 1 pouce (0,75 à 2,5 cm), selon la taille du renfort.

Avantageusement, ladite découpe peut aménager ladite zone de passage selon une forme allongée dans une direction sensiblement parallèle à la direction longitudinale générale dudit bord longitudinal.

Cette zone de passage allongée permet également de ne pas délimiter un point de passage déterminé du cordon spermatique au travers du renfort, et donc de ne pas imposer une position déterminée de mise en place du renfort.

De préférence, ladite découpe consiste en une échancrure délimitée par des bords respectivement sensiblement perpendiculaire et sensiblement parallèle à la direction longitudinale générale dudit bord longitudinal.

Le rabat est de préférence relié à ladite première pièce selon une ligne sensiblement perpendiculaire à la direction longitudinale générale dudit bord longitudinal, cette ligne étant en particulier une ligne de couture.

Avantageusement, le rabat présente une extension venant, lorsque ce rabat est placé contre ladite première pièce, recouvrir ladite deuxième pièce.

Cette extension contribue à prévenir efficacement le risque d'une récidive de la hernie.

Avantageusement, le rabat est maintenu contre ladite première pièce au moyen d'une pièce de matériau agrippant fixée sur ladite première pièce ou sur ledit rabat, cette pièce de matériau agrippant comprenant des picots propres à venir s'insérer dans les fibres dudit rabat ou de ladite première pièce et à accrocher ces fibres.

Ce matériau agrippant permet le maintien fiable du rabat le long de ladite première pièce.

La présente invention est maintenant décrite par référence au dessin annexé, dans lequel
la figure 1 est une vue à plat, en éclaté, des différentes pièces qui constituent le renfort qu'elle concerne;
la figure 2 est une vue en perspective de ce renfort;
la figure 3 en est une vue en perspective après implantation, le site d'implantation étant représenté de manière très simplifiée;
la figure 4 en est une vue en coupe selon la ligne IV-IV de la 5 figure 3, et
la figure 5 en est une vue selon selon la ligne V-V de la figure 3.

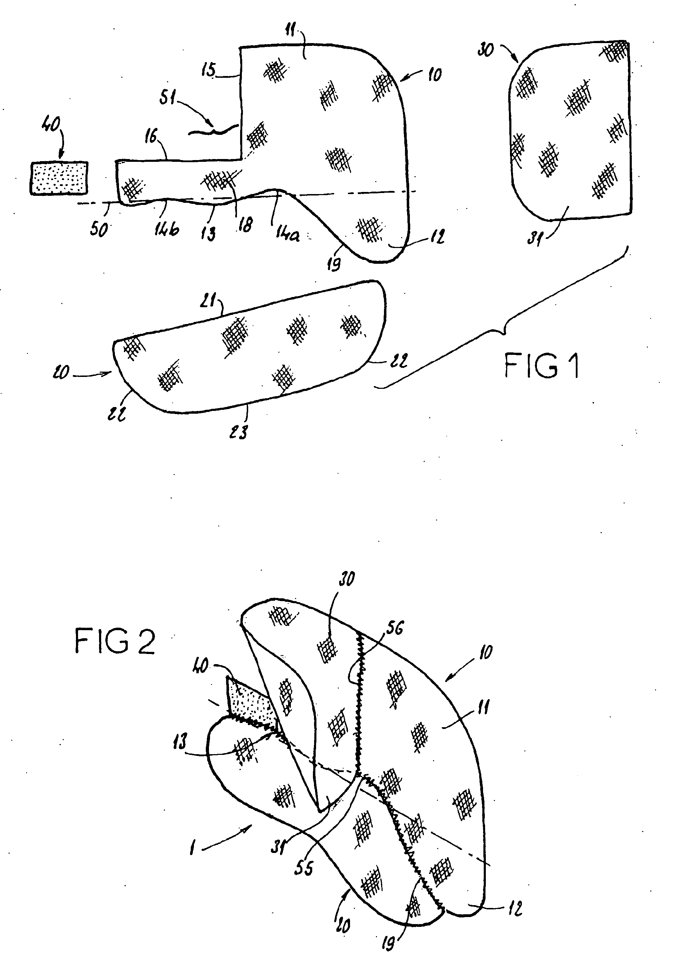

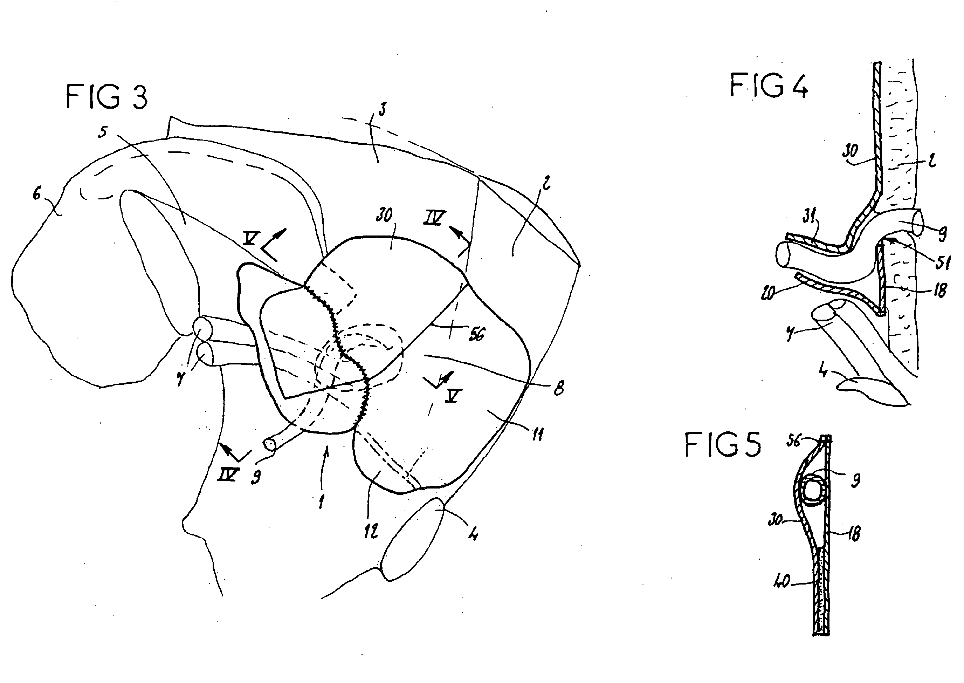

Les figures 2 et 3 représentent un renfort pariétal anatomique 1 permettant le traitement d'une hernie inguinale. Il s'agit, dans l'exemple représenté, d'un renfort de traitement d'une hernie inguinale du côté gauche, un renfort de forme symétrique étant utilisé pour traiter une hernie inguinale du côté droit.

La figure 3 montre, outre le renfort 1, la région inguinale extrapéritonéale, vue de l'intérieur de l'abdomen vers l'extérieur de celui-ci. Il peut y être reconnu le muscle grand droit 2, le muscle transverse 3, le tubercule pubien 4, le muscle psoas 5, l'aile iliaque 6, les vaisseaux spermatiques et iliaques 7, l'orifice interne 8 du canal inguinal et le cordon spermatique 9. Le péritoine, le fascia transversalis et le ligament de Cooper ne sont pas représentés pour la clarté du dessin.

Ainsi que le montrent les figures 1 et 2, le renfort 1 comprend une première pièce 10, une deuxième pièce 20, un rabat 30, et une pièce 40 en matériau agrippant.

La pièce 10 est en un matériau poreux et flexible, relativement rigide comparativement au matériau de la pièce 20, par exemple en un tissu de fils multifilaments de polyester, à simple épaisseur. Elle présente une partie principale 11 destinée à venir reposer contre la paroi musculaire, notamment contre les muscles grand droit 2 et transverse 3, et une partie secondaire 12, minoritaire en surface, destinée à venir recouvrir l'extrémité supérieure de l'os pubien et le ligament de Cooper.

Le bord longitudinal 13 de la partie principale 11 duquel se projette la partie secondaire 12 présente une forme ondulée définissant un creux 14a proche de la partie 12, conformé pour contourner les vaisseaux spermatiques et iliaques 7, et un creux 14b distant de la partie 12, conformé pour s'adapter à la forme du muscle psoas 5.

La pièce 10 comprend également une échancrure délimitée par des bords 15 et 16 respectivement sensiblement perpendiculaire et sensiblement parallèle à la direction longitudinale générale 50 du bord longitudinal 13. Ces bords 15 et 16 aménagent une zone de passage 51 du cordon spermatique 9 au-delà de la pièce 10, située à distance du bord longitudinal 13 et ayant une forme allongée dans une direction sensiblement parallèle à ladite direction longitudinale générale 50. La largeur de la zone 18 de la pièce 10 s'étendant entre le bord longitudinal 13 et le bord 16 peut aller de 0,3 à 1 pouce (0,75 à 2,5 cm), selon la taille du renfort 1.

La deuxième pièce 20 est également en un matériau poreux et flexible, plus souple que le matériau constituant la pièce 10, par exemple en un tricot ou un tissu de fils polyester multifilaments ayant 1 à 2 mm d'épaisseur.

Cette pièce 20 présente un bord longitudinal rectiligne 21, des bords d'extrémités arrondis 22 et un bord longitudinal 23 opposé au bord 21 sensiblement rectiligne. Ainsi que cela apparaît sur la figure 2, la pièce 20 est assemblée à la pièce 10 le long de la totalité du bord longitudinal 13 et du bord latéral 19 de la partie secondaire 12 consécutif à ce bord longitudinal 13, par une couture 55 réalisée légèrement en retrait de ces bords 13 et 19. La pièce 20 présente ainsi une forme ondulée qui suit les ondulations du bord 13 et du bord 19, adaptée à la forme des structures anatomiques inférieures de l'espace inguinal à traiter, qu'elle recouvre partiellement.

Le rabat 30 est en un matériau poreux et flexible, plus souple que le matériau constituant la pièce 10. Il peut notamment être réalisé dans le même matériau que celui constituant la pièce 20.

Ce rabat 30 est relié à la pièce 10 selon une ligne de couture 56 sensiblement perpendiculaire à la direction longitudinale générale 50 du bord longitudinal 13, et est dimensionné de manière à recouvrir l'échancrure aménagée dans la pièce 10, la zone 18, et, par une extension libre 31 qui le prolonge, la partie de la pièce 20 s'étendant en face de lui.

La pièce 40 comprend une structure de base tissée ou tricotée et inclut des picots aggripants propres à s'insérer dans la structure du rabat 30 et à agripper les fils et fibres de celui-ci.

Cette pièce 40 est dimensionnée pour n'occuper qu'une partie de la zone 18 et est fixée à cette zone 18 du côté longitudinal de la pièce 10 opposée à celui sur lequel se trouve la partie 12, à distance du bord 15. Elle permet de maintenir le rabat 30 plaqué contre la pièce 10.

Le renfort 1 est destiné à être mis en place dans l'espace inguinal à traiter notamment par voie postérieure en utilisant une technique de laparoscopie. Cette technique, bien connue en elle-même et donc non décrite en détails, comprend l'aménagement d'un espace extrapéritonéal entre le fascia transversalis et les muscles grand droit et transversal par insufflation d'air et séparation du péritoine et de la paroi abdominale puis mise en place d'un ou plusieurs trocarts de travail, dont un permet l'introduction du renfort 1.

Après introduction, ce dernier est déployé dans ledit espace extrapéritonéal; la partie 11 de la pièce 10 vient reposer contre les muscles grand droit et transversal et la partie 12 vient reposer contre l'os pubien en recouvrant le ligament de Cooper, le bord inférieur de la partie 11 venant reposer contre les vaisseaux 7 en les contournant et prendre appui contre le muscle psoas 5 ; la pièce 20 vient quant à elle recouvrir ces vaisseaux 7 et une partie du muscle psoas 5.

Le rabat 30 est soulevé pour permettre l'engagement du cordon spermatique 9 le long de la zone 18 puis est rabattu contre la pièce 10 de manière à être agrippé par la pièce 40.

Il apparaît sur la figure 4 que, dans cette position rabattue du rabat 30, le cordon spermatique 9 rentre dans le renfort 1 en étant reçu contre la zone 18 et sort du renfort 1 par ladite zone de passage 51, au-delà du bord 16. Les zones par lesquelles le cordon spermatique 9 rentre au travers du renfort 1 et sort au-delà de ce renfort sont ainsi mutuellement décalées et distantes, de sorte que le cordon spermatique 9 traverse le renfort 1 selon un parcours en chicane.

Ce parcours se fait entre la zone 18 d'une part et le rabat 30 d'autre part, qui recouvre parfaitement le cordon spermatique 9, particulièrement au niveau de la zone de passage 51.

Le renfort 1 permet ainsi de parfaitement prévenir toute récidive de hernie.

De plus, comme cela apparaît sur la figure 5, la zone de passage 51 et le rabat 30 ne déterminent pas une zone précise d'engagement du cordon spermatique 9 au travers du renfort 1. Cette zone 51 et ce rabat 30 n'obligent donc pas à un positionnement déterminé du renfort 1 dans le site d'implantation et permettent de positionner le bord médial du renfort 1 au-delà de la ligne médiane si nécéssaire. La mise en place du renfort 1 s'en trouve facilitée, et le rabat 30 facilite lui-même l'engagement du cordon spermatique 9 au travers du renfort 1.

Ainsi qu'il apparaît de ce qui précède, l'invention apporte des améliorations déterminantes aux renforts homologues de la technique antérieure.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées.

## Revendications

1. - Renfort pariétal anatomique (1) pour le traitement d'une hernie inguinale, comprenant une première pièce (10) et une deuxième pièce (20) assemblées l'une à l'autre, ladite première pièce (10) présentant une partie principale (11) destinée à venir reposer contre la paroi musculaire, notamment les muscles grand droit (2) et transversal (3), et une partie secondaire (12), minoritaire en surface, destinée à venir recouvrir l'extrémité supérieure de l'os pubien et le ligament de Cooper, le bord longitudinal (13) de ladite partie principale (11) à partir duquel se projette ladite partie secondaire (12) ayant une forme plus ou moins ondulée, adaptée à celle des structures inguinales inférieures, à savoir notamment les vaisseaux spermatiques et iliaques (7) et le muscle psoas (5), ladite deuxième pièce (20) étant assemblée à ladite première pièce (10) le long d'une partie ou de la totalité dudit bord longitudinal (13) et du bord latéral de ladite partie secondaire (12) consécutif à ce bord longitudinal (13), et étant destinée à épouser la forme générale desdites structures inguinales inférieures,
renfort **caractérisé** ou ce que ladite première pièce (10) comprend une découpe aménageant une zone de passage (51) du cordon spermatique (9) à travers elle située à distance dudit bord longitudinal (13), et comprend un rabat (30) relié à elle, dimensionné de manière à s'étendre près dudit bord longitudinal (13) et à recouvrir largement la zone (18) de ladite première pièce (10) s'étendant entre ledit bord longitudinal (13) et ladite zone de passage (51), ce rabat (30) pouvant être soulevé par rapport à ladite première pièce (10) pour l'engagement du cordon spermatique (9) entre ladite première pièce (10) et lui-même et pouvant être rabattu contre ladite première pièce (10) pour maintenir ce cordon (9) entre cette première pièce (10) et lui-même.

2. - Renfort pariétal anatomique (1) selon la revendication 1, dans lequel la largeur de ladite zone (18) de la première pièce (10) s'étendant entre ledit bord longitudinal (13) et ladite zone de passage (51), peut aller de 0,3 à 1 pouce (0,75 à 2,5 cm), selon la taille du renfort (1).

3. - Renfort pariétal anatomique (1) selon la revendication 1, dans lequel ladite découpe aménage ladite zone de passage (51) selon une forme allongée dans une direction sensiblement parallèle à la direction longitudinale générale (50), dudit bord longitudinal (13).

4. - Renfort pariétal anatomique (1) selon la revendication 1, dans lequel ladite découpe consiste en une échancrure délimitée par des bords (15,16) respectivement sensiblement perpendiculaire et sensiblement parallèle à la direction longitudinale générale (50) dudit bord longitudinal (13).

5. - Renfort pariétal anatomique (1) selon la revendication 1, dans lequel le rabat (30) est relié à ladite première pièce (10) selon une ligne sensiblement perpendiculaire à la direction longitudinale générale (50) dudit bord longitudinal (13).

6. - Renfort pariétal anatomique (1) selon la revendication 5, dans lequel ladite ligne est une ligne de couture (56).

7. - Renfort pariétal anatomique (1) selon la revendication 1, dans lequel le rabat (30) présente une extension venant, lorsque ce rabat (30) est placé contre ladite première pièce (10), recouvrir ladite deuxième pièce (20).

8. - Renfort pariétal anatomique (1) selon la revendication 1, dans lequel le rabat (30) est maintenu contre ladite première pièce (10) au moyen d'une pièce (40) de matériau agrippant fixée sur ladite première pièce ou sur ledit rabat (30), cette pièce (40) de matériau agrippant comprenant des picots propres à venir s'insérer dans les fibres dudit rabat (30) ou de ladite première pièce (10) et à accrocher ces fibres.

## Claims

1. An anatomical wall reinforcement (1) for the treatment of an inguinal hernia, comprising a first piece (10) and a second piece (20) which are assembled with one another, said first piece (10) having a main part (11) intended to rest against the muscle wall, in particular the rectus (2) and transverse (3) muscles, and a secondary part (12), which is smaller in area, intended to cover the upper end of the pubic bone and the Cooper's ligament, the longitudinal edge (13) of said main part (11), from which said secondary part (12) projects, having a more or less corrugated shape suited to that of the lower inguinal structures, namely in particular the spermatic and iliac vessels (7) and the psoas muscle (5), said second piece (20) being assembled with said first piece (10) along a part or all of said longitudinal edge (13) and the side edge of said secondary part (12) consecutive to this longitudinal edge (13), and being intended to match the overall shape of said lower inguinal structures,
in which reinforcement said first piece (10) comprises a cutout forming a passage zone (51), located at a distance from said longitudinal edge, for the spermatic cord (9) to pass through it, and comprises a flap (30) which is joined to it and is dimensioned so as to extend close to said longitudinal edge (13) and to broadly cover the zone (18) of said first piece (10) extending between said longitudinal edge (13) and said passage zone (51), this flap (30) being raisable in relation to said first piece (10) in order to engage the spermatic cord (9) between said first piece (10) and itself and being foldable against said first piece (10) in order to hold this cord (9) between this first piece (10) and itself.

2. The anatomical wall reinforcement (1) as claimed in claim 1, in which the width of said zone (18) of the first piece (10) extending between said longitudinal edge (13) and said passage zone (51) can range from 0.3 to 1 inch (0.75 to 2.5 cm), depending on the size of the reinforcement (1).

3. The anatomical wall reinforcement (1) as claimed in claim 1, in which said cutout forms said passage zone (51) with a shape that is elongate in a direction substantially parallel to the overall longitudinal direction (50) of said longitudinal edge (13).

4. The anatomical wall reinforcement (1) as claimed in claim 1, in which said cutout consists of a notch bounded by edges (15, 16) respectively substantially perpendicular and substantially parallel to the overall longitudinal direction (50) of said longitudinal edge (13).

5. The anatomical wall reinforcement (1) as claimed in claim 1, in which the flap (30) is joined to said first piece (10) along a line substantially perpendicular to the overall longitudinal direction (50) of said longitudinal edge (13).

6. The anatomical wall reinforcement (1) as claimed in claim 5, in which said line is a seam line (56).

7. The anatomical wall reinforcement (1) as claimed in claim 1, in which the flap (30) has an extension which, when this flap (30) is placed against said first piece (10), covers said second piece (20).

8. The anatomical wall reinforcement (1) as claimed in claim 1, in which the flap (30) is held against said first piece (10) by means of a piece of gripping material fixed on said first piece or on said flap (30), this piece (40) of gripping material comprising spikes capable of being inserted into the fibers of said flap (30) or of said first piece (10), and capable of hooking these fibers.

## Patentansprüche

1. Anatomische parietale Verstärkung (1) für die Behandlung eines Leistenbruches, mit einem ersten Stück (10) und einem zweiten Stück (20), die miteinander zusammengefügt sind, wobei das erste Stück (10) einen Hauptteil (11), der dazu bestimmt ist, gegen die Muskelwand, insbesondere den geraden Bauchmuskel (2) und queren Bauchmuskel (3) zu liegen zu kommen, und einen Nebenteil (12) aufweist, der hinsichtlich der Oberfläche kleiner ist, und der dazu bestimmt ist, das obere Ende des Schambeins und das Cooper-Band zu bedecken, wobei der Längsrand (13) des Hauptteils (11), von dem aus sich der Nebenteil (12) erstreckt, eine mehr oder weniger gewellte Form aufweist, die an diejenige der unteren Leistenstrukturen, nämlich insbesondere den Samen- und Darmgefäßen (7) und den Lendenmuskel (5) angepasst ist, wobei das zweite Stück (20) mit dem ersten Stück (10) entlang eines Teils des oder des gesamten Längsrandes (13) und des Seitenrandes des Nebenteils (12), der sich an diesen Längsrand (13) anschließt, zusammengefügt ist, und dazu bestimmt ist, sich an die allgemeine Form der unteren Leistenstrukturen anzuschmiegen,
wobei die Verstärkung **dadurch gekennzeichnet ist, dass** das erste Stück (10) einen Schnitt aufweist, der durch es hindurch eine Durchgangszone (51) für den Samenleiter (9) ausspart, die sich im Abstand von dem Längsrand (13) befindet, und eine mit ihm verbundene Umschlagklappe (30) aufweist, die derart bemessen ist, dass sie sich nahe des Längsrandes (13) erstreckt und die Zone (18) des ersten Stückes (10) weit bedeckt, die sich zwischen dem Längsrand (13) und der Durchgangszone (51) erstreckt, wobei die Umschlagklappe (30) bezüglich des ersten Stückes (10) für das In-Eingriff-Kommen des Samenleiters (9) zwischen dem ersten Stück (10) und ihr selbst angehoben werden kann und gegen das erste Stück (10) umgeschlagen werden kann, um den Samenleiter (9) zwischen dem ersten Stück (10) und ihr selbst zu halten.

2. Anatomische parietale Verstärkung (1) nach Anspruch 1, bei der die Breite der Zone (18) des ersten Stückes (10), die sich zwischen dem Längsrand (13) und der Durchgangszone (51) erstreckt, gemäß der Größe der Verstärkung (1) von 0,3 bis 1 Zoll (0,75 bis 2,5 cm) reichen kann.

3. Anatomische parietale Verstärkung (1) nach Anspruch 1, bei der der Schnitt die Durchgangszone (51) gemäß einer länglichen Form in einer zur allgemeinen Längsrichtung (50) des Längsrandes (13) etwa parallelen Richtung ausspart.

4. Anatomische parietale Verstärkung (1) nach Anspruch 1, bei der der Schnitt aus einem Ausschnitt besteht, der von Rändern (15, 16) begrenzt ist, die etwa senkrecht bzw. etwa parallel zur allgemeinen Längsrichtung (50) des Längsrandes (13) verlaufen.

5. Anatomische parietale Verstärkung (1) nach Anspruch 1, bei der die Umschlagklappe (30) mit dem ersten Stück (10) entlang einer etwa senkrecht zur allgemeinen Längsrichtung (50) des Längsrandes (13) verlaufenden Linie verbunden ist.

6. Anatomische parietale Verstärkung (1) nach Anspruch 5, bei der die Linie eine Nahtlinie (56) ist.

7. Anatomische parietale Verstärkung (1) nach Anspruch 1, bei der die Umschlagklappe (30) eine Erweiterung aufweist, die, wenn die Umschlagklappe (30) gegen das erste Stück (10) liegt, das zweite Stück (20) bedeckt.

8. Anatomische parietale Verstärkung (1) nach Anspruch 1, bei der die Umschlagklappe (30) mittels eines Stückes (40) aus greifendem Material gehalten ist, das an dem ersten Stück oder an der Umschlagklappe (30) fixiert ist, wobei das Stück (40) aus greifendem Material Zähnchen aufweist, die geeignet sind, sich in die Fasern der Umschlagklappe (30) oder des ersten Stückes (10) einzugreifen und an diesen Fasern hängen zu bleiben.
